# EUROPÄISCHE PATENTSCHRIFT

(11) **EP 1 569 710 B1**
(45) Veröffentlichungstag und Bekanntmachung des Hinweises auf die Patenterteilung: **27.05.2009**
(21) Anmeldenummer: 03782349.9
(22) Anmeldetag: 09.12.2003
(51) Int. Cl.: A61M 15/00

(54) **Inhalationstherapievorrichtung**
Inhalation therapy device
Dispositif de traitement par inhalation

(30) Priorität: 09.12.2002 DE 10257381
(43) Veröffentlichungstag der Anmeldung: 07.09.2005
(73) Patentinhaber: PARI Pharma GmbH, 82319 Starnberg (DE)
(72) Erfinder: WALDNER, Robert, 86971 Peiting (DE); MUNDENBRUCH, Daniela, 81247 München (DE); HETZER, Uwe, 81369 München (DE); URICH, Markus, 81739 München (DE)
(74) Vertreter: HOFFMANN EITLE
(86) Internationale Anmeldenummer: PCT/EP2003/013959
(87) Internationale Veröffentlichungsnummer: WO 2004/052436

(56) Entgegenhaltungen:
- WO-A-98/32479
- DE-A- 10 022 795
- DE-C1- 19 953 317
- US-A- 6 062 212
- US-B1- 6 412 481

## Beschreibung

Die Erfindung betrifft eine Inhalationstherapievorrichtung mit einem Aerosolmembrangenerator und einer Mischkammer sowie einem Einatemventil.

Mit Inhalationstherapievorrichtungen dieser Art können medikamenthaltige Flüssigkeiten, flüssige Medikamente oder andere therapeutisch einsetzbare Flüssigkeiten, beispielsweise Salzlösungen, vernebelt werden, um dem Patienten ein Aerosol für die Inhalation darzubieten.

DE 199 53 317 A beschreibt eine derartige Inhalationstherapievorrichtung mit einem Membranaerosolgenerator, dessen Membran durch einen Schwingungsgenerator in Schwingungen versetzt wird, wodurch eine auf der einen Seite der Membran anstehende Flüssigkeit durch die Membran hindurch vernebelt und in eine Mischkammer hinein abgegeben wird. In der Mischkammer durchmischt sich das Aerosol während des Einatmungsvorgangs mit Umgebungsluft, die der Mischkammer durch einen um einen Flüssigkeitsvorratsbehälter gebildeten Ringspalt zugeführt wird. In dem Ringspalt ist ein ebenes kreisförmiges Einatemventil vorgesehen, das den Ringspalt während der Einatemphase freigibt und während der Ausatemphase verschließt.

Wenn bei dem bekannten Therapievernebler der Patient in die Vorrichtung hinein ausatmet, gelangt zwar ein großer Teil der Atemluft über ein in einem Mundstück der Vorrichtung vorgesehenes Ausatemventil unmittelbar in Umgebung, jedoch sind auch der Aerosolmembrangenerator und der Bereich des Flüssigkeitsvorratsbehälters einem Teil der Atemluft ausgesetzt. Es kommt auf diese Weise zu unerwünschten Verunreinigungen des Aerosolgenerators.

Das von der Erfindung zu lösende Problem besteht vor dem Hintergrund dieses Standes der Technik darin, eine Inhalationstherapievorrichtung anzugeben, bei der das Einatemventil derart gestaltet ist, dass das Risiko einer Verunreinigung des Aerosolmembrangenerators durch die Atemluft des Patienten während der Ausatemphasen verringert ist.

Dieses Problem wird erfindungsgemäß gelöst durch eine Inhalationstherapievorrichtung mit einem Aerosolmembrangenerator, mit einem Flüssigkeitsvorratsbehälter, in den eine therapeutisch einsetzbare Flüssigkeit einfüllbar ist, mit einer Membran, die auf einer Seite mit dem Flüssigkeitsbehälter derart in Verbindung steht, dass eine in den Flüssigkeitsvorratsbehälter eingefüllte Flüssigkeit in Berührung mit einer Seite der Membran gelangt, und mit einem Schwingungsgenerator für die Erzeugung von Schwingungen, durch die eine in den Flüssigkeitsvorratsbehälter eingefüllte Flüssigkeit durch Öffnungen der Membran hindurch auf der anderen Seite der Membran zu einem Aerosol zerstäubt wird. Ferner weist die erfindungsgemäße Inhalationstherapievorrichtung eine Mischkammer, in die hinein der Aerosolmembrangenerator das Aerosol erzeugt, und ein Einatemventil auf, das in Einatemphasen den Zustrom von Umgebungsluft in die Mischkammer zulässt und in Ausatemphasen das Austreten des Aerosols aus der Mischkammer verhindert und das einen Wandabschnitt der Mischkammer bildet. Das Einatemventil wiederum umfasst einen Aerosoldurchlass, durch den das von dem Membrangenerator erzeugte Aerosol in die Mischkammer gelangt, der mit einem Abschnitt auf einer Oberfläche des Aerosolmembrangenerators die Membran längs zumindest einer Dichtlinie umschließend angeordnet ist und der sich in die Mischkammer hinein öffnend erstreckt, zumindest eine Atemluftdurchtrittsöffnung, die in einem Bereich um den Aerosoldurchlass angeordnet ist, und ein Ventilelement, das im Bereich um den Aerosoldurchlass derart angeordnet ist, dass das Ventilelement die zumindest eine Atemluftdurchtrittsöffnung in Ausatemphasen verschließt und in Einatemphasen freigibt.

Durch die räumlich benachbarte Anordnung des Aerosoldurchlasses, der Atemluftdurchtrittsöffnung und des Ventilelements wird ein Einatemventil geschaffen, das sich als Wandabschnitt der Mischkammer eignet und dadurch vor dem Membranvernebler angeordnet werden kann. Die außerhalb der Mischkammer liegenden Teile der Gesamtvorrichtung sind deshalb besser vor Verunreinigungen geschützt. Der Aerosoldurchlass stellt nicht nur sicher, dass eine Abdichtung der Mischkammer im Bereich um die Membran herum erfolgt, sondern unterstützt auch wirksam die Ausbreitung des Aerosols von der Membran in die Mischkammer hinein. Dabei ist vorteilhaft, dass durch den Aerosoldurchlass eine zuverlässige Abdichtung an der Oberfläche des Aerosolgenerators um die Membran herum gewährleistet werden kann. Ferner wird bei dem erfindungsgemäßen Inhalationstherapiegerät die Atemluft bezogen auf den Aerosoldurchlass außen geführt und schirmt dadurch das durch den Aerosoldurchlass in die Mischkammer eintretende Aerosol gewissermaßen ab, so dass eine unerwünschte Impaktion der Aerosolpartikel bzw. -tröpfchen an der Wand der Mischkammer verringert wird.

Vorzugsweise sind mehrere Atemlufdurchtrittsöffnungen vorhanden, die von dem einen oder von mehreren getrennten oder miteinander verbundenen Ventilelementen verschlossen bzw. freigegeben werden.

Für die einfache Halterung des Ventilelements ist eine umlaufende Nut vorgesehen.

Um das Risiko von Beschädigungen zu verringern und die Halterung zu verbessern, weist das Ventilelement am Rand für die Halterung in der umlaufenden Nut eine Verdickung auf.

In einer bevorzugten Ausgestaltung ist der Aerosoldurchlass rohrartig und das Ventilelement ringförmig ausgebildet. Das Ventilelement nimmt dann den rohrartige Aerosoldurchlass in der Ringöffnung auf. Vorzugsweise ist in diesem Fall die umlaufende Nut für die Aufnahme des Ventilelements in der äußeren Mantelfläche des rohrartigen Aerosoldurchlasses vorgesehen.

In einer bevorzugten Ausgestaltung wird der rohrartige Aerosoldurchlass durch eine zylindrische Hülse gebildet, an deren Mantelfläche ein die Atemluftdurchtrittsöffnungen aufnehmender Bereich vorgesehen ist, der sich im wesentlichen senkrecht zur Längsachse der Hülse erstreckt. Vorzugsweise ist die Hülse konzentrisch zu der Membran angeordnet. In Anpassung an diese Gestaltung ist das Ventilelement kreisringförmig ausgebildet und nimmt die zylindrische Hülse in der Ringöffnung auf. Entsprechend weist das ringförmige Ventilelement am Rand der Ringöffnung für die Halterung an der zylindrischen Hülse eine Verdickung auf. Zur Fixierung ist vorteilhafterweise die umlaufende Nut für die Aufnahme des Randes der Ringöffnung des Ventilelements in der Mantelfläche der zylindrischen Hülse vorgesehen.

Um Beschädigungen zu vermeiden und die Abdichtung der Mischkammer im Bereich der Membran zu unterstützen, weist der Aerosoldurchlass in dem der Oberfläche des Aerosolmembrangenerators zugewandten Bereich eine Verdickung auf.

In einer Grundform erstrecken sich die Atemluftdurchtrittsöffnungen im wesentlichen parallel zu dem Aerosoldurchlass. In einer vorteilhaften Gestaltung verlaufen die Atemluftdurchtrittsöffnungen jedoch spiralartig, um der durch die Öffnungen strömenden Luft einen Drall aufzuprägen.

Grundsätzlich ist eine Gestaltung zu bevorzugen, bei der die Atemluftdurchtrittsöffnungen als Kreisringabschnitte gebildet werden.

Das Einatemventil ist insgesamt vorzugsweise mit einem Randabschnitt ausgestaltet, der für die Halterung des Einatemventils, insbesondere für das Einklemmen zwischen Aerosolgenerator und Mischkammer ausgestaltet ist.

Vorzugsweise sind die Atemluftdurchtrittsöffnungen derart schräg verlaufend ausgestaltet, dass die Atemluft von der Fixierungsstelle des Ventilelements weg geführt wird. Dadurch wird die Auslösung des Ventils zu Beginn der Einatemphasen unterstützt und eine verbesserte Strömungsführung unterstützt, die zu geringen Depositionsraten insbesondere an den Mischkammerwänden führt.

Geringere Depositionsraten werden auch durch eine Gestaltung erzielt, bei der die Atemluftdurchtrittsöffnungen allseitig um den Aerosoldurchlass herum vorgesehen sind.

In einer speziellen Ausgestaltung ist der Bereich der Atemluftdurchtrittsöffnungen im wesentlichen in einer Ebene mit der Membran angeordnet.

Bevorzugt ist das Ventilelement aus einem elastischen Material hergestellt. Auch das Einatemventil ist vorzugsweise aus einem elastischen Material hergestellt.

Die Erfindung wird im Folgenden anhand eines Ausführungsbeispiels und unter Bezugnahme auf die Zeichnungen genauer erläutert. In den Zeichnungen zeigt:
- Fig. 1: eine geschnittene Ansicht eines Ausführungsbeispiels einer erfindungsgemäßen Inhalationsvorrichtung;
- Fig. 2: in einer Ausschnittsansicht den Bereich der Atemluftdurchtrittsöffnungen mit spiralartig ausgebildeten Öffnungen;
- Fig. 3A und 3B: perspektivische Ansichten eines erfindungsgemäßen Einatemventils;
- Fig. 4: ein weiteres Ausführungsbeispiel einer erfindungsgemäßen Inhalationsvorrichtung;
- Fig. 5: eine Abwandlung des Ausführungsbeispiels gemäß Figur 4;
- Fig. 6: ein weiteres Ausführungsbeispiel einer erfindungsgemäßen Inhalationsvorrichtung;
- Fig. 7: eine Abwandlung des Ausführungsbeispiels gemäß Figur 6; und
- Fig. 8: ein weiteres Ausführungsbeispiel einer erfindungsgemäßen Inhalationsvorrichtung.

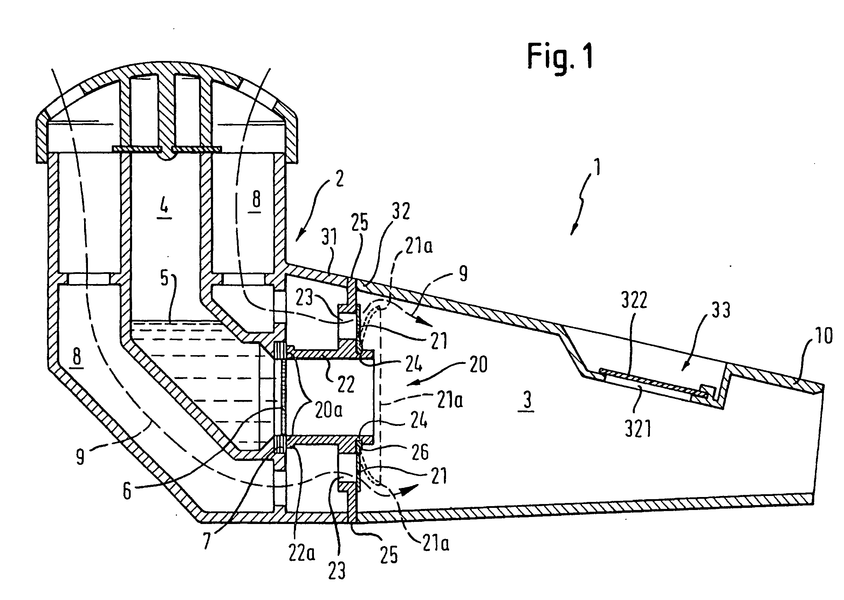

Die Figur 1 zeigt ein Ausführungsbeispiel eines erfindungsgemäßen Inhalationstherapiegerätes 1, das einen Aerosolmembrangenerator 2 und eine Mischkammer 3 aufweist. In dem gezeigten Ausführungsbeispiel sind die beiden Komponenten als getrennte Einheiten ausgelegt, die miteinander auf geeignete Weise verbunden werden, so dass beide Komponenten als funktionsfähige Einheit sicher gemeinsam gehandhabt werden können.

Der Aerosolgenerator 2 des Ausführungsbeispiels aus Figur 1 umfasst einen Flüssigkeitsvorratsbehälter 4, in den eine medikamenthaltige Flüssigkeit 5 einfüllbar ist, und eine Membran 6, die den Flüssigkeitsvorratsbehälter 4 an einer geöffneten Fläche abschließt. Dadurch steht die Membran 6 auf einer Seite mit dem Flüssigkeitsbehälter 4 derart in Verbindung, dass die in den Flüssigkeitsvorratsbehälter eingefüllte Flüssigkeit 5 die eine Seite der Membran 6 berührt. An der Membran 6 ist ein Schwingungsgenerator 7, beispielsweise ein Piezo-Element, angeordnet, durch den die Membran 6 in Schwingungen versetzt wird, wenn der Schwingungsgenerator 7 angesteuert wird. In dem hier gezeigten Ausführungsbeispiel sind Membran 6 und Schwingungsgenerator 7 rotationssymmetrisch ausgelegt, so dass der Schwingungsgenerator 7 die Membran konzentrisch umgibt.

Wird der Schwingungsgenerator 7 angeregt, also beispielsweise das Piezo-Element mit einer Wechselspannung beaufschlagt, wird die Membran 6 in Schwingungen versetzt, so dass die in den Flüssigkeitsvorratsbehälter eingefüllte und an der Membran 6 anstehende Flüssigkeit 5 durch Öffnungen in der Membran hindurch auf die andere Seite der Membran 6 gefördert und dort zu einem Aerosol zerstäubt wird. Das Aerosol wird in die Mischkammer 3 hinein abgegeben.

Bei dem in Figur 1 gezeigten Ausführungsbeispiel ist ferner ein Mundstück 10 vorgesehen, das in dem gezeigten Beispiel einstückig mit der Mischkammer 3 ausgebildet ist, das aber in einer abgewandelten Ausführungsform auch von der Mischkammer 3 trennbar ausgestaltet werden kann. Über das Mundstück 10 inhaliert der Patient, das von dem Aerosolmembrangenerator 2 erzeugte und in die Mischkammer 3 abgegebene Aerosol, wenn er durch das Mundstück einatmet.

Während der Einatemphase strömt Umgebungsluft durch Zuluftkanäle 8, die in dem Aerosolmembrangenerator 2 ausgebildet sind, in das Therapiegerät und gelangt in die Mischkammer 3; der Strömungsverlauf der Zuluft ist in Figur 1 mit den gestrichelten Pfeillinien 9 angedeutet. Dabei ist ein Ventilelement 21, das im weiter unten noch ausführlich beschrieben wird, angehoben, was in Figur 1 der gestrichelten Position 21a entspricht.

Erfindungsgemäß ist zur Steuerung/Kontrolle der Zuluft ein Einatemventil 20 vorgesehen, das in Einatemphasen den Zustrom von Umgebungsluft in die Mischkammer zulässt, das aber in Ausatemphasen verhindert; dass die ausgeatmete Atemluft aus der Mischkammer heraus zu dem Aerosolmembrangenerator 2 gelangt. Damit verhindert das erfindungsgemäße Einatemventil 20, dass in Ausatemphasen das Aerosol mit der Atemluft aus der Mischkammer transportiert wird und dass Atemluft in den Aerosolmembrangenerator 2 strömt. Dadurch wird in dem Aerosolgenerator 2 der Bereich um den Flüssigkeitsbehälter 4 herum, in dem sich oftmals auch andere Komponenten, beispielsweise elektrische Anschlüsse für die Ansteuerung des Piezo-Kristalls befinden, vor Atemluft und damit vor Verunreinigungen geschützt, die mit der Atemluft in das Therapiegerät gelangen können. Somit schützt das erfindungsgemäße Einatemventil 20 das Innere des Aerosolgenerators 2 während der Ausatemphasen, indem es die Mischkammer 3 zum Aerosolmembrangenerator 2 hin verschließt.

Die Ausatemluft wird auf andere Weise aus der Mischkammer 3 bzw. dem Mundstück 10 geführt, beispielsweise über ein an sich bekanntes Mundstückventil 32, das eine Mundstückventilöffnung 321 und ein Mundstückventilelement 322 umfasst. Auf die Funktionsweise des Mundstückventils wird hier aber nicht näher eingegangen.

Erfindungsgemäß bildet das Einatemventil 20, wie in Figur 1 gezeigt, einen Wandabschnitt der Mischkammer 3, in dem es einen offen Wandbereich der Mischkammer 3 verschließend ergänzt. Bei der in Figur 1 gezeigten Ausführung ist das Einatemventil 20 zwischen Aerosolmembrangenerator 2 und Mischkammer 3 eingeklemmt.

Das Einatemventil 20 umfasst einen Aerosoldurchlass 22, der es dem Aerosol gestattet, von der Membran in die Mischkammer zu gelangen. Außerdem umfasst das Einatemventil 20 eine oder mehrere Atemluftdurchtrittsöffnungen 23, durch die in den Eintatemphasen Zuluft in die Mischkammer gelangt und die in den Ausatemphasen verschlossen sind. Dazu besitzt das Einatemventil 20 ein oder mehrere Ventilelemente 21. Dieser grundsätzliche Aufbau wird im folgenden anhand des Ausführungsbeispiels gemäß Figur 1 exemplarisch näher erläutert.

Bei dem gezeigten Ausführungsbeispiel umfasst das Einatemventil 20 eine zylindrische Hülse 22, die den Aerosoldurchlass darstellt, durch den das von der Membran abgegebene Aerosol in die Mischkammer 3 strömt. Die Hülse ist an der Membran 6 ausgerichtet und vorzugsweise konzentrisch zur Membran 6 angeordnet. Die Hülse 22 liegt mit einer Stirnseite auf einer Oberfläche des Membrangenerators 2 auf, die die Membran 6 umgibt. Dadurch wird eine Abdichtung der Mischkammer im Bereich um die Membran 6 herum geschaffen, die verhindert, dass in Ausatemphasen Atemluft an der Membran 6 vorbei strömt.

Grundsätzlich betrachtet weist der Aerosoldurchlass 22 zu diesem Zweck einen Bereich auf, der auf einer Oberfläche des Aerosolmembrangenerators 2 angeordnet ist, so dass die Membran längs zumindest einer Dichtlinie 20a umschlossen ist. Auf diese Weise stellt der Aerosoldurchlass 22 des Einatemventils 20 sicher, dass einerseits das Aerosol von der Membran 6 in die Mischkammer 3 gelangen kann und dass andererseits eine Abdichtung der Mischkammer 3 im Bereich des Aerosolmembrangenerators 2 erfolgt.

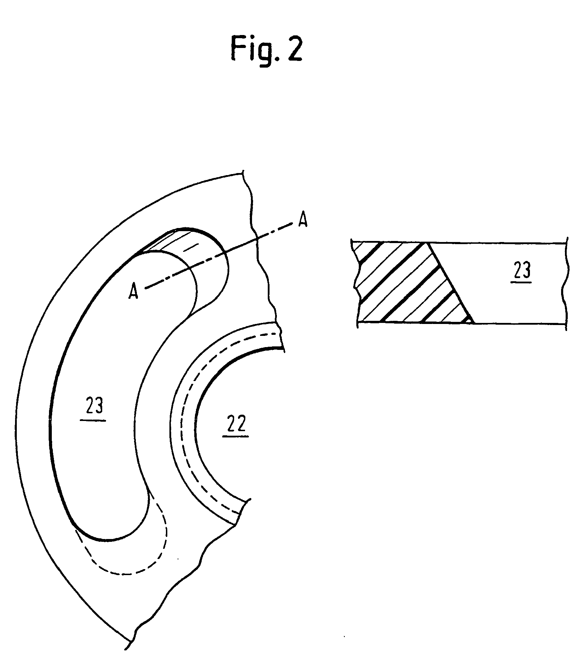

Wie sich aus den Figur 1 ergibt, ist bei dem gezeigten Ausführungsbeispiel um die Hülse 22 herum ein Bereich vorgesehen, in dem Atemluftdurchtrittsöffnungen 23 vorgesehen sind. Der die Atemluftdurchtrittsöffnungen enthaltende Bereich erstreckt sich im wesentlichen senkrecht zur Längsachse der Hülse 22. Die Atemluftdurchtrittsöffnungen 23 liegen konzentrisch zur zylindrischen Hülse 22 und sind in Form von Kreisringsegmenten gebildet, von denen eines in Figur 2 gezeigt ist.

Die in Figur 2 gezeigt Ausführung der Atemluftdurchtrittsöffnungen 23 ist in sofern bemerkenswert, als die Öffnungen, abweichend von einer Grundform, bei der die Atemluftdurchtrittsöffnungen 23 im wesentlichen parallel zum Aerosoldurchlass 22 verlaufen, derart schräg ausgebildet sind, dass sich eine spiralförmige Gestaltung ergibt. Diese Ausbildung ist in Figur 2 anhand des Schnitts entlang der Linie A-A verdeutlicht. Durch die spiralartige Schräggestaltung der Atemluftdurchtrittsöffnungen 23 wird der Atemluft, die durch diese Öffnungen strömt, ein Drall aufgeprägt, der dazu führt, dass die Atemluft das durch den Aerosoldurchlass strömende Aerosol in der Mischkammer außen umgibt und gewissermaßen einschließt. Dadurch wird die Gefahr einer Impaktion der Aerosolpartikel auf der Innenwand der Mischkammer 3 weiter reduziert.

Auf der der Mischkammer zugewandten Seite des Bereichs der Atemluftdurchtrittsöffnungen 23 ist ein Ventilelement 21 angeordnet, das bei dem gezeigten Ausführungsbeispiel kreisringförmig und flach ist und das die zylindrische Hülse 22 in seiner zentrischen Ringöffnung aufnimmt. Figur 1 zeigt neben der Stellung, in der das Ventilelement 21 die Atemluftdurchtrittsöffnungen 23 verschließt, in gestrichelter Darstellung das Ventilelement 21a in angehobener Stellung, d.h. während der Einatemphasen.

Zur Halterung des Ventilelements 21 weist die Hülse 22 in der äußeren Mantelfläche vorzugsweise eine umlaufende Nut 24 auf, in der der innere Rand der Ringöffnung des Ventilelements 21 angeordnet ist. Der Rand der Ringöffnung ist dabei mit einer Verdickung 26 versehen. Dadurch wird nicht nur eine sichere Halterung des Ventilelements 21 in der Nut 24 gewährleistet, sondern auch der innere Rand der Ringöffnung gegen Beschädigungen beim Aufstecken geschützt.

Bei dem in Figur 1 gezeigten Ausführungsbeispiel ist das Einatemventil 20 zwischen einem Wandabschnitt 31 des Aerosolgenerators 2 und einem Wandabschnitt 31 der Mischkammer 3 angeordnet und vorzugsweise derart gehaltert, beispielsweise eingeklemmt, dass das Einatemventil 20 sicher fixiert und dessen Aerosoldurchlass 22 exakt positioniert ist. Das Einatemventil 20 weist dazu einen äußeren Randabschnitt 25 auf, der für die Halterung/Fixierung an dem Aerosolgenerator 2 und/oder der Mischkammer 3 ausgebildet ist. Besonders vorteilhaft ist das in Figur 1 gezeigte Ausführungsbeispiel auch deshalb, weil das Einatemventil 20 zur Abdichtung der Verbindungsstelle zwischen Aerosolgenerator 2 und Mischkammer 3 eingesetzt wird. Dazu ist der Randabschnitt 25 des Einatemventils 20 an die stirnseitigen Querschnitte des Aerosolgenerators 2 und der Mischkammer 3 angepasst.

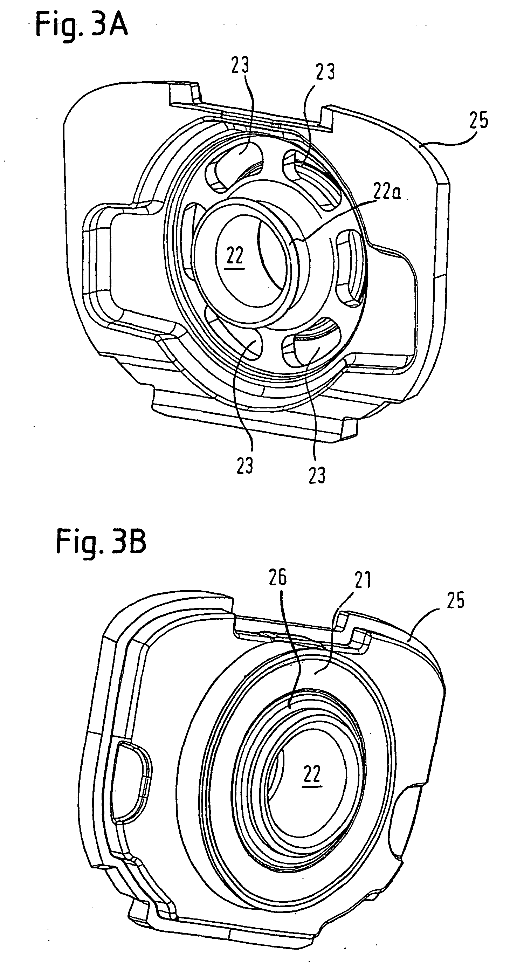

Die Figuren 3A und 3B zeigen, dass die Anpassung des Randabschnitts 25 an den Querschnitt des Membranaerosolgenerators 2 / der Mischkammer 3 sehr weit gehen kann, wobei aber stets problemlos ein Übergang zu dem erfindungsgemäßen Bereich mit den Atemluftdurchtrittsöffnungen 23 geschaffen werden kann. Durch diese Art der Anpassung wird erreicht, dass das für Abdichtungszwecke grundsätzlich geeignete Material des Einatemventils 20 auch für die Abdichtung des Übergangs zwischen Aerosolgenerator 2 und Mischkammer 3 herangezogen wird.

Daraus ergibt sich ferner, dass das erfindungsgemäße Einatemventil 20 vorzugsweise nur aus zwei Teilen aufgebaut ist. Denn der eine Teil umfasst in einem Stück die Hülse 22, den Bereich der Atemluftdurchtrittsöffnungen 23, den Übergangsbereich zum Randabschnitt 25 und den Randabschnitt 25 selbst; den anderen Teil bildet das Ventilelement 21.

Aus den Figuren 1 und 3A ergibt sich ferner, dass das der Membran zugewandte stirnseitige Ende der zylindrischen Hülse 22 vorzugsweise eine umlaufende Verdickung 22a aufweist. Dadurch wird die Gefahr einer Beschädigung des stirnseitigen Endes der Hülse verringert. In Figur 3B, die eine perspektivische Ansicht eines erfindungsgemäßen Einatemventils von der Seite der Mischkammer 3 her zeigt, ist die Verdickung 26 an der inneren Kante der Ringöffnung des Ventilelements 21 deutlich erkennbar.

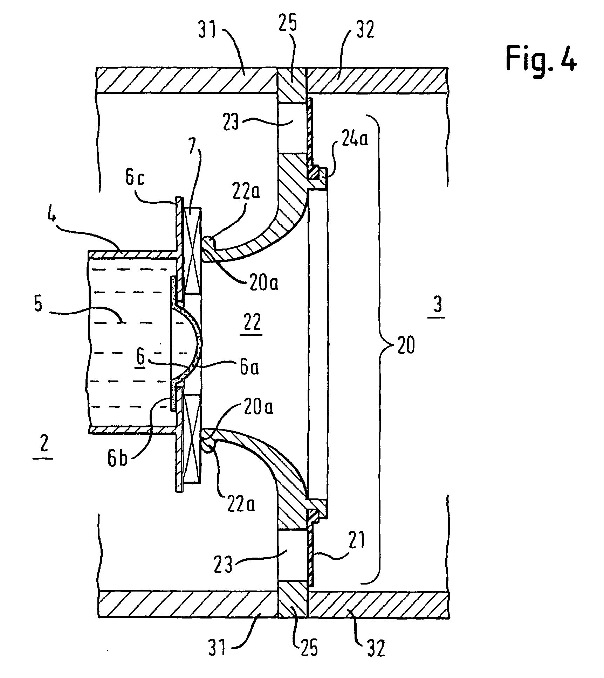

Figur 4 zeigt ein weiteres Ausführungsbeispiel eines erfindungsgemäßen Einatemventils 20 in einer Darstellung, in der Teile des Aerosolmembrangenerators 2 und der Mischkammer 3 nicht wiedergegeben sind; diesbezüglich wird auf Figur 1 verwiesen. In Figur 4 ist der Vorratsbehälter 4 und die Flüssigkeit 5 erkennbar, die an die Membran 6 angrenzt. Die Membran 6 ist bei diesem Ausführungsbeispiel mit einem Kalottenabschnitt 6a versehen, der sich zur Mischkammer 3 hin wölbt. Ein Befestigungsabschnitt 6b der Membran 6 dient der Befestigung der Membran an einem Träger 6c, an dem der Schwingungsgenerator 7, beispielsweise das Piezo-Element, befestigt ist. Hinsichtlich des Funktionsprinzips des Aerosolgenerators dieses Ausführungsbeispiels wird auf die Ausführungen weiter oben in Bezug auf Figur 1 verwiesen.

Das erfindungsgemäße Einatemventil 20 dieses Ausführungsbeispiels umfasst einen Aerosoldurchlass 22, der es dem von der Membran abgegebenen Aerosol gestattet, in die Mischkammer 3 -zu gelangen. Der Aerosoldurch-lass 22 ist bei diesem Ausführungsbeispiel im wesentlichen ein flaches trichterförmiges Gebilde. Der Aerosoldurchlass 22 weist einen Bereich auf, der auf einer Oberfläche des Aerosolmembrangenerators 2, gemäß Figur 4 eine Oberfläche des Schwingungsgenerators 7, aufliegt und die Membran 6 längs einer Dichtlinie 20a umgibt. Dieser Bereich des Aerosoldurchlasses 22 ist vorzugsweise mit einer Verdickung 22a ausgestattet. Bei dem in Figur 4 gezeigten Ausführungsbeispiel ist erkennbar, dass der Aerosoldurchlass 22, die sich ergebende Dichtlinie 20a und die Verdickung 22a vorzugsweise rotationssymmetrisch sind.

Bei dem Aerosoldurchlass 22 sind eine oder mehrere Atemluftdurchtrittsöffnungen 23 in einem dafür vorgesehenen Bereich ausgebildet. Diese Öffnungen werden zur Mischkammer 3 hin von einem Ventilelement 21 abgedeckt, so dass die Öffnungen während der Einatemphasen freigegeben und während der Ausatemphasen verschlossen sind. Die Einzelheiten des Ventilelements 21 entsprechen denen des Ventilelements des zuvor geschilderten Ausführungsbeispiels, so dass an dieser Stelle auf die Erläuterungen oben verwiesen werden kann.

Zur sicheren Halterung ist an dem Aerosoldurchlass 22 gemäß Figur 4 ein Vorsprung 24a vorgesehen, der bei rotationssymmetrischer Gestaltung des Aerosoldurchlasses kreisförmig. In diesem Vorsprung 24a ist die umlaufende Nut 24 ausgebildet, in der das Ventilelement 21, vorzugsweise mit der Verdickung 26, fixiert ist.

Auch bei dem Ausführungsbeispiel gemäß Figur 4 schließt sich an den Bereich mit den Atemluftdurchtrittsöffnungen 23 ein Randbereich 25 an, der zur Halterung des Einatemventils 20 dient, indem der Rand 25 beispielsweise zwischen einer Wand 31 des Aerosolmembrangenerators 2 und einer Wand 32 der Mischkammer 3 eingeklemmt wird.

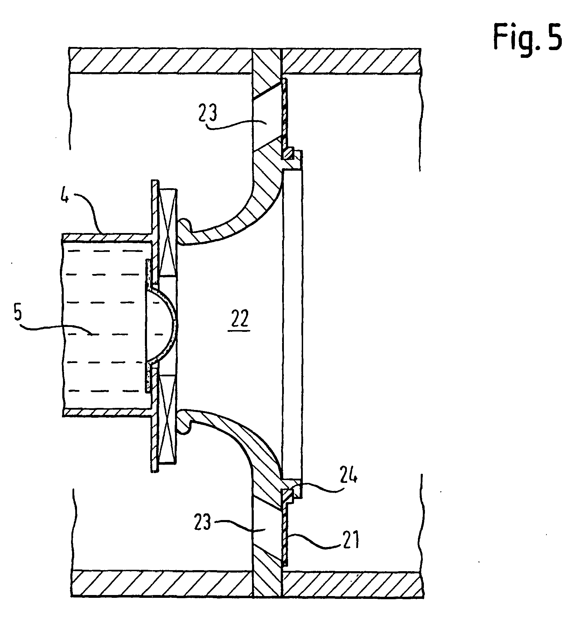

Figur 5 zeigt eine Abwandlung des Ausführungsbeispiels gemäß Figur 4, auf dessen Beschreibung auch für Figur 5 Bezug genommen wird. Zur Verdeutlichung der Abwandlung sind in Figur 5 nur solche Bezugszeichen angegeben, die in unmittelbarer Beziehung zu der Abwandlung stehen.

Abweichend von dem Ausführungsbeispiel gemäß Figur 4 sind bei dem Ausführungsbeispiel gemäß Figur 5 die Atemluftöffnungen 23 nach außen schräg verlaufend ausgebildet. Mit anderen Worten, die Atemluftdurchtrittsöffnungen 23 erstrecken von der fixierten Stelle des Ventilelements 21 weg, das in der Nut 24 gehaltert ist. Dadurch wird die Atemluft in Bezug auf den Aerosoldurchlass 22 nach außen und damit zu dem freien Ende des sich abhebenden Abschnitts des Ventilelements 21 geführt, was zu einer günstigeren Strömung in der Mischkammer 3 und zu einer geringeren Auslösekraft für das Ventilelement 21 führt.

Zusätzlich zu der in Figur 5 gezeigten Schrägstellung der Atemluftöffnungen 23 kann eine spiralartige Ausgestaltung, die weiter oben mit Bezug auf Figur 2 beschrieben wurde, auch bei dem Ausführungsbeispielen gemäß den Figuren 4 und 5 vorgesehen werden. Bei der Ausgestaltung gemäß Figur 5 wird die Atemluft dadurch nicht nur nach außen geführt, sondern ihr wird zusätzlich ein Drall aufgeprägt.

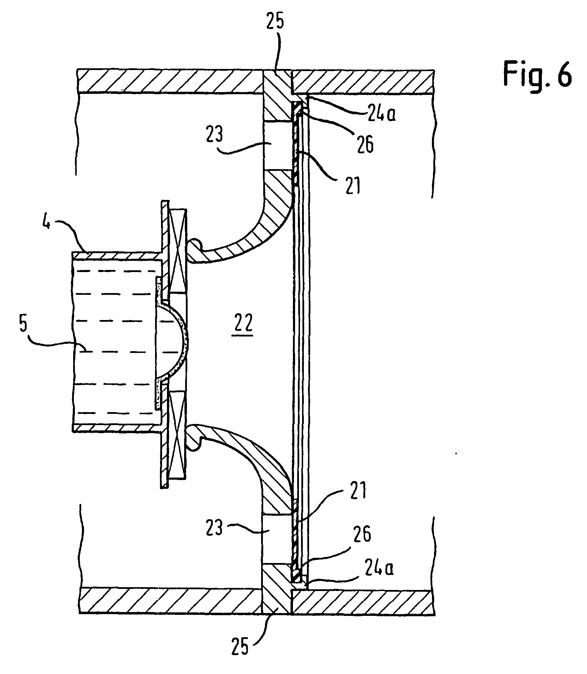

Figur 6 zeigt eine Abwandlung des Ausführungsbeispiels gemäß Figur 4, auf dessen Beschreibung auch für Figur 6 Bezug genommen wird. Zur Verdeutlichung der Abwandlung sind in Figur 6 nur solche Bezugszeichen angegeben, die in unmittelbarer Beziehung zu der Abwandlung stehen.

Abweichend von dem Ausführungsbeispiel gemäß Figur 4 ist bei dem Ausführungsbeispiel gemäß Figur 6 der Vorsprung 24a in Bezug auf den Aerosoldurchlass 22 außen, vorzugsweise in der Nähe zum Randbereich 25, angeordnet. In diesem Vorsprung 24a ist die umlaufende Nut 24 so ausgebildet, dass sie sich zum Aerosoldurchlass 22 öffnet. Dementsprechend ist das Ventilelement 21 an einer äußeren Kante in der Nut 24 gehaltert und weist dazu vorzugsweise eine Verdickung 26 an der äußeren Kante auf. Das Ventilelement 21 erstreckt sich mit seinem flexiblen Abschnitt zum Aerosoldurchlass 22 und deckt dadurch die Atemluftdurchtrittsöffnungen 23 ab. Im übrigen entspricht die Funktionsweise des Ventilelements 21 dieses Ausführungsbeispiels vollständig den zuvor geschilderten Ausführungsbeispielen.

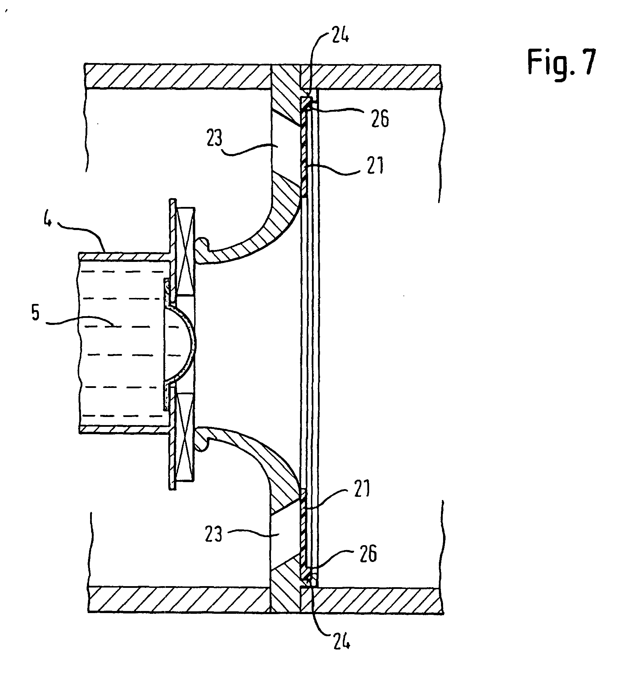

Figur 7 zeigt eine Abwandlung des Ausführungsbeispiels gemäß Figur 6, auf dessen Beschreibung auch für Figur 7 Bezug genommen wird. Zur Verdeutlichung der Abwandlung sind in Figur 7 nur solche Bezugszeichen angegeben, die in unmittelbarer Beziehung zu der Abwandlung stehen.

Abweichend von dem Ausführungsbeispiel gemäß Figur 6 sind bei dem Ausführungsbeispiel gemäß Figur 7 die Atemluftöffnungen 23 nach innen schräg verlaufend ausgebildet. Mit anderen Worten, die Atemluftdurchtrittsöffnungen 23 erstrecken von der fixierten Stelle des Ventilelements 21 weg, das in der Nut 24 gehaltert ist. Dadurch wird die Atemluft in Bezug auf den Aerosoldurchlass 22 nach innen und damit zu dem freien Ende des sich abhebenden Abschnitts des Ventilelements 21 geführt, was zu einer günstigeren Strömung in der Mischkammer 3 und zu einer geringeren Auslösekraft für das Ventilelement 21 führt.

Zusätzlich zu der in Figur 7 gezeigten Schrägstellung der Atemluftöffnungen 23 kann eine spiralartige Ausgestaltung, die weiter oben mit Bezug auf Figur 2 beschrieben wurde, auch bei dem Ausführungsbeispielen gemäß den Figuren 6 und 7 vorgesehen werden. Bei der Ausgestaltung gemäß Figur 7 wird die Atemluft dadurch nicht nur nach innen geführt, sondern ihr wird zusätzlich ein Drall aufgeprägt.

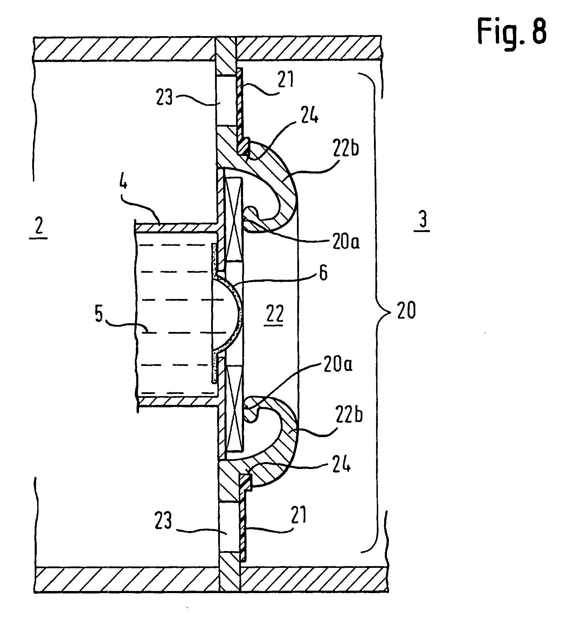

Figur 8 zeigt ein weiteres Ausführungsbeispiel eines erfindungsgemäßen Einatemventils 20 in einer Darstellung, in der Teile des Aerosolmembrangenerators 2 und der Mischkammer 3 nicht wiedergegeben sind; diesbezüglich wird auf Figur 1 verwiesen. Bei diesem Ausführungsbeispiel ist der Aerosoldurchlass 22 sehr flach ausgebildet; der Bereich mit den Atemluftdurchtrittsöffnungen 23 ist im wesentlichen in einer Ebene zu der Membran 6 des Aerosolmembrangenerators 2 angeordnet. Dennoch besitzt auch der Aerosoldurchlass 22 dieses Einatemventils 20 erfindungsgemäß einen Bereich, der auf einer Oberfläche des Aerosolmembrangenerators 2 aufliegt und der die Membran längs zumindest einer Dichtlinie 20a umgibt, wodurch die Abdichtung der Mischkammer um die Membran herum sichergestellt ist. Der Erfindung entsprechend ist das Ventilelement 21 so ausgebildet und angeordnet, dass es die Atemluftdurchtrittsöffnungen 23 während der Ausatemphasen verschließt und während der Einatemphasen freigibt. Zwischen dem Aerosoldurchlass 22 und dem Bereich der Atemluftdurchtrittsöffnungen 23 ist ein Übergangsbereich 22b ausgebildet, in dem vorzugsweise die umlaufende Nut 24 vorgesehen ist. In Anlehnung an Figur 6 oder 7 kann aber auch außen ein die Nut aufnehmender Vorsprung vorgesehen sein. Die Atemluftdurchtrittsöffnungen 23 können auch bei diesem Ausführungsbeispiel in Anlehnung an die Figuren 5 und 7 entsprechend der Fixierungsstelle des Ventilelements 21 nach außen oder nach innen schräg verlaufen und in Anlehnung an Figur 2 spiralartig ausgebildet sein.

## Patentansprüche

1. Inhalationstherapievorrichtung mit
a. einem Aerosolmembrangenerator (2),
i. mit einem Flüssigkeitsvorratsbehälter (4), in den eine therapeutisch einsetzbare Flüssigkeit (5) einfüllbar ist,
ii. mit einer Membran (6), die auf einer Seite mit dem Flüssigkeitsbehälter (4) derart in Verbindung steht, dass eine in den Flüssigkeitsvorratsbehälter eingefüllte Flüssigkeit (5) in Berührung mit einer Seite der Membran (6) gelangt, und
iii. mit einem Schwingungsgenerator (7) für die Erzeugung von Schwingungen, durch die eine in den Flüssigkeitsvorratsbehälter eingefüllte Flüssigkeit (5) durch Öffnungen der Membran (6) hindurch auf der anderen Seite der Membran zu einem Aerosol zerstäubt wird,
b. einer Mischkammer (3), in die hinein der Aerosolmembrangenerator (2) das Aerosol erzeugt, und
c. einem Einatemventil (20, 21), das in Einatemphasen den Zustrom von Umgebungsluft in die Mischkammer (3) zulässt und in Ausatemphasen das Austreten des Aerosols aus der Mischkammer (3) verhindert und das einen Wandabschnitt der Mischkammer (3) bildet, **gekennzeichnet durch**
i. einen Aerosoldurchlass (22), **durch** den das von dem Membrangenerator erzeugte Aerosol in die Mischkammer (3) gelangt, der mit einem Abschnitt auf einer Oberfläche des Aerosolmembrangenerators (2) die Membran (3) längs zumindest einer Dichtlinie (20a) umschließend angeordnet ist und der sich in die Mischkammer (3) hinein öffnend erstreckt,
ii. zumindest eine Atemluftdurchtrittsöffnung (23), die im Bereich um den Aerosoldurchlass (22) angeordnet ist, und
iii. ein Ventilelement (21), das im Bereich um den Aerosoldurchlass (22) derart angeordnet ist, dass das Ventilelement (21) die zumindest eine Atemluftdurchtrittsöffnung (23) in Ausatemphasen verschließt und in Einatemphasen freigibt.

2. Inhalationstherapievorrichtung nach Anspruch 1, **dadurch gekennzeichnet, dass** mehrere Atemluftdurchtrittsöffnungen (23) vorgesehen sind.

3. Inhalationstherapievorrichtung nach Anspruch 1 oder 2, **dadurch gekennzeichnet, dass** eine umlaufende Nut (24) für die Halterung des Ventilelements (21) vorgesehen ist.

4. Inhalationstherapievorrichtung nach Anspruch 3, **dadurch gekennzeichnet, dass** für die Halterung in der umlaufenden Nut (24) das Ventilelements (21) eine Verdickung (26) aufweist.

5. Inhalationstherapievorrichtung nach einem der Ansprüche 1 bis 4, **dadurch gekennzeichnet, dass** der Aerosoldurchlass (22) rohrartig und das Ventilelement (21) ringförmig ausgebildet ist und das Ventilelement (21) den rohrartige Aerosoldurchlass (22) in der Ringöffnung aufnimmt.

6. Inhalationstherapievorrichtung nach Anspruch 5, **dadurch gekennzeichnet, dass** die umlaufende Nut (24) für die Aufnahme des Ventilelements (21) in der äußeren Mantelfläche des rohrartigen Aerosoldurchlasses (22) vorgesehen ist.

7. Inhalationstherapievorrichtung nach einem der Ansprüche 5 oder 6, **dadurch gekennzeichnet, dass** der rohrartige Aerosoldurchlass gebildet wird durch eine zylindrische Hülse (22), an deren äußerer Mantelfläche ein die Atemluftdurchtrittsöffnungen (23) aufnehmender Bereich vorgesehen ist, der sich im wesentlichen senkrecht zur Längsachse der Hülse erstreckt.

8. Inhalationstherapievorrichtung nach Anspruch 7, **dadurch gekennzeichnet, dass** die zylindrische Hülse (22) konzentrisch zu der Membran (6) angeordnet ist.

9. Inhalationstherapievorrichtung nach einem der Ansprüche 7 oder 8, **dadurch gekennzeichnet, dass** das Ventilelement (21) kreisringförmig ausgebildet ist und die zylindrische Hülse (22) in der Ringöffnung aufnimmt.

10. Inhalationstherapievorrichtung nach einem der vorangegangenen Ansprüche, **dadurch gekennzeichnet, dass** der Aerosoldurchlass (22) in dem der Membran zugewandten Bereich eine Verdickung (22a) aufweist.

11. Inhalationstherapievorrichtung nach einem der vorangegangenen Ansprüche, **dadurch gekennzeichnet, dass** sich die eine oder mehreren Atemluftdurchtrittsöffnungen (23) im wesentlichen parallel zu dem Aerosoldurchlass (22) erstrecken.

12. Inhalationstherapievorrichtung nach einem der vorangegangenen Ansprüche 1 bis 9, **dadurch gekennzeichnet, dass** die eine oder mehreren Atemluftdurchtrittsöffnungen (23) in Bezug auf den Aerosoldurchlass (22) spiralartig verlaufen.

13. Inhalationstherapievorrichtung nach einem der vorangegangenen Ansprüche, **dadurch gekennzeichnet, dass** die eine oder mehreren Atemluftdurchtrittsöffnungen (23) als Kreisringabschnitte bzw. -segmente gebildet werden.

14. Inhalationstherapievorrichtung nach einem der vorangegangenen Ansprüche, **dadurch gekennzeichnet, dass** das Einatemventil (20) einen Randabschnitt (25) aufweist, der für die Halterung des Einatemventils (20), insbesondere für das Einklemmen zwischen einer Wand (31) des Aerosolgenerators (2) und einer Wand (32) der Mischkammer (3) ausgestaltet ist.

15. Inhalationstherapievorrichtung nach einem der vorangegangenen Ansprüche, **dadurch gekennzeichnet, dass** die eine oder mehreren Atemluftdurchtrittsöffnungen (23) allseitig um den Aerosoldurchlass (22) vorgesehen sind.

16. Inhalationstherapievorrichtung nach einem der vorangegangenen Ansprüche, **dadurch gekennzeichnet, dass** die Atemluftdurchtrittsöffnungen (23) derart schräg verlaufend ausgestaltet sind, dass die Atemluft von der Fixierungsstelle des Ventilelements (21) weg geführt wird.

17. Inhalationstherapievorrichtung nach einem der vorangegangenen Ansprüche, **dadurch gekennzeichnet, dass** der Bereich der einen oder mehreren Atemluftdurchtrittsöffnungen (23) im wesentlichen in einer Ebene mit der Membran (6) angeordnet ist.

18. Inhalationstherapievorrichtung nach einem der vorangegangenen Ansprüche, **dadurch gekennzeichnet, dass** das Ventilelement (21) aus einem elastischen Material hergestellt ist.

19. Inhalationstherapievorrichtung nach einem der vorangegangenen Ansprüche, **dadurch gekennzeichnet, dass** das Einatemventil (20) aus einem elastischen Material hergestellt ist.

## Claims

1. Inhalation therapy device having
a. an aerosol membrane generator (2)
i. having a liquid storage container (4) into which a liquid (5) usable for therapy can be charged,
ii. having a membrane (6) connected on one side to the liquid container (4) in such a way that a liquid (5) charged into the liquid storage container comes into contact with one side of the membrane (6), and
iii. having a vibration generator (7) for generating vibrations by means of which a liquid (5) charged into the liquid storage container is atomised to form an aerosol on the other side of the membrane through openings in the membrane (6),
b. a mixing chamber (3) into which the aerosol membrane generator (2) generates the aerosol, and
c. an inhalation valve (20, 21) which in inhalation phases allows the flow of ambient air into the mixing chamber (3) and in exhalation phases prevents the escape of the aerosol from the mixing chamber (3) and which forms a wall section of the mixing chamber (3), **characterised by**
i. an aerosol outlet (22) through which the aerosol generated by the membrane generator comes into the mixing chamber (3), which is arranged by a section on a surface of the aerosol membrane generator (2) enclosing the membrane (3) along at least one sealing line (20a) and which extends opening into the mixing chamber,
ii. at least one inhaled air passage opening (23) arranged in the region of the aerosol outlet (22), and
iii. a valve element (21) arranged in the region around the aerosol outlet (22) in such a way that the valve element (21) seals the at least single inhaled air passage opening (23) in exhalation phases and releases it in inhalation phases.

2. Inhalation therapy device according to claim 1, **characterised in that** a plurality of inhaled air passage openings (23) is provided.

3. Inhalation therapy device according to claim 1 or 2, **characterised in that** an all-round groove (24) is provided for mounting the valve element (21).

4. Inhalation therapy device according to claim 3, **characterised in that** for mounting in the all-round groove (24) the valve element (21) comprises a bulge (26).

5. Inhalation therapy device according to any of claims 1 to 4, **characterised in that** the aerosol outlet (22) is of tubular construction and the valve element (21) of annular construction and the valve element (21) accommodates the tubular aerosol outlet (22) in the annular opening.

6. Inhalation therapy device according to claim 5, **characterised in that** the all-round groove (24) is provided for accommodating the valve element (21) in the outer jacket surface of the tubular aerosol outlet (22).

7. Inhalation therapy device according to one of claims 5 or 6, **characterised in that** the tubular aerosol outlet is formed by a cylindrical sleeve (22) on whose outer jacket surface a region accommodating the inhaled air passage openings (23) is provided which extends substantially perpendicular to the longitudinal axis of the sleeve.

8. Inhalation therapy device according to claim 7, **characterised in that** the cylindrical sleeve (22) is arranged concentrically with respect to the membrane (6).

9. Inhalation therapy device according to one of claims 7 or 8, **characterised in that** the valve element (21) is of annular construction and accommodates the cylindrical sleeve (22) in the annular opening.

10. Inhalation therapy device according to any of the preceding claims, **characterised in that** the aerosol outlet (22) in the region facing towards the membrane has a bulge (22a).

11. Inhalation therapy device according to any of the preceding claims, **characterised in that** the single or several inhaled air passage openings (23) extend substantially parallel to the aerosol outlet (22).

12. Inhalation therapy device according to any of the preceding claims 1 to 9, **characterised in that** the single or several inhaled air passage openings (23) run in the shape of a spiral with respect to the aerosol outlet (22).

13. Inhalation therapy device according to any of the preceding claims, **characterised in that** the single or several inhaled air passage openings (23) are formed as circular sections or segments.

14. Inhalation therapy device according to any of the preceding claims, **characterised in that** the inhalation valve (20) has a peripheral section (25) which is designed for fixing the inhalation valve (20), in particular for clamping it between a wall (31) of the aerosol generator (2) and a wall (32) of the mixing chamber (3).

15. Inhalation therapy device according to any of the preceding claims, **characterised in that** the single or several inhaled air passage openings (23) are provided on all sides around the aerosol outlet (22).

16. Inhalation therapy device according to any of the preceding claims, **characterised in that** the inhaled air passage openings (23) are designed to run obliquely in such a way that the inhaled air is carried away from the fixing point of the valve element (21).

17. Inhalation therapy device according to any of the preceding claims, **characterised in that** the region of the single or several inhaled air passage openings (23) is arranged substantially in a plane with the membrane (6).

18. Inhalation therapy device according to any of the preceding claims, **characterised in that** the valve element (21) is manufactured from an elastic material.

19. Inhalation therapy device according to any of the preceding claims, **characterised in that** the inhalation valve (20) is manufactured from an elastic material.

## Revendications

1. Dispositif de traitement par inhalation, comprenant
a. un générateur d'aérosol à membrane (2),
i. avec un récipient de réserve de liquide (4), dans lequel un liquide (5) utilisable à des fins thérapeutiques est susceptible d'être introduit,
ii. avec une membrane (6), reliée, sur un côté, au récipient à liquide (4), de manière qu'un liquide (5), introduit dans le récipient de réserve de liquide, vienne en contact avec un côté de la membrane (6), et
iii. avec un générateur de vibrations (7) pour la génération de vibrations, au moyen desquelles le liquide (5), introduit dans le récipient de réserve de liquide, passant par des ouvertures de la membrane (6), est pulvérisé de l'autre côté de la membrane, en un aérosol,
b. une chambre de mélange (3), dans laquelle le générateur de vibrations (7) produit l'aérosol, et
c. une soupape d'inspiration (20, 21) qui, dans les phases d'inspiration, permet l'arrivée d'écoulement d'air ambiant dans la chambre de mélange (3) et, dans les phases d'expiration, empêche la sortie de l'aérosol hors de la chambre de mélange (3), et forme un tronçon de paroi de la chambre de mélange (3), **caractérisé par** :
i. un passage à aérosol (22), à travers lequel l'aérosol, produit par le générateur à membrane, arrive dans la chambre de mélange (3), en étant disposé en entourant la membrane (6), par un tronçon, le long d'au moins une ligne d'étanchéité (20a), sur une surface du générateur d'aérosol à membrane (2), et s'étendant en s'ouvrant dans la chambre de mélange (3),
ii. au moins un ouverture de passage d'air respiratoire (23), disposée dans la zone situe autour du passage à aérosol (22), et
iii. un élément de soupape (21), disposé dans la zone située autour du passage à aérosol (22), de manière que, dans des phases d'expiration, l'élément de soupape (21) ferme la au moins une ouverture de passage d'air respiratoire (23) et, dans des phases d'inspiration, il la libère.

2. Dispositif de traitement par inhalation selon la revendication 1, **caractérisé en ce que** plusieurs ouvertures de passage d'air respiratoire (23) sont prévues.

3. Dispositif de traitement par inhalation selon la revendication 1 ou 2, **caractérisé en ce qu'**une gorge (24) de pourtour est prévue pour assurer la fixation de l'élément de soupape (21).

4. Dispositif de traitement par inhalation selon la revendication 3, **caractérisé en ce que** l'élément de soupape (21) présente un épaississement (26), pour assurer la fixation dans la gorge (24) de pourtour.

5. Dispositif de traitement par inhalation selon l'une des revendications 1 à 4, **caractérisé en ce que** le passage à aérosol (22) est de conformation tubulaire et l'élément de soupape (21) est conformé en anneau, et l'élément de soupape (21) loge le passage à aérosol (22) tubulaire dans l'ouverture annulaire.

6. Dispositif de traitement par inhalation selon la revendication 5, **caractérisé en ce que** la gorge (24) de pourtour est prévue pour recevoir l'élément de soupape (21) dans la surface d'enveloppe extérieure du passage à aérosol (22) tubulaire.

7. Dispositif de traitement par inhalation selon l'une des revendications 5 ou 6, **caractérisé en ce que** le passage à aérosol tubulaire est formé par une douille (22) cylindrique, sur la surface d'enveloppe extérieure de laquelle est prévue une zone, recevant les ouvertures de passage d'air respiratoire (23), qui s'étend sensiblement perpendiculairement à l'axe longitudinal de la douille.

8. Dispositif de traitement par inhalation selon la revendication 7, **caractérisé en ce que** la douille (22) cylindrique est disposée concentriquement à la membrane (6).

9. Dispositif de traitement par inhalation selon l'une des revendications 7 ou 8, **caractérisé en ce que** l'élément de soupape (21) est de configuration en anneau de cercle et reçoit la douille (22) cylindrique dans l'ouverture de l'anneau.

10. Dispositif de traitement par inhalation selon l'une des revendications précédentes, **caractérisé en ce que** le passage à aérosol (22) présente un épaississement (22a) dans la zone tournée vers la membrane.

11. Dispositif de traitement par inhalation selon l'une des revendications précédentes, **caractérisé en ce que** la une ou plusieurs ouvertures de passage d'air respiratoire (23) s'étendent sensiblement parallèlement au passage à aérosol (22).

12. Dispositif de traitement par inhalation selon l'une des revendications 1 à 9, **caractérisé en ce que** la une ou plusieurs ouvertures de passage d'air respiratoire (23) s'étendent en spirale, par rapport au passage à aérosol (22).

13. Dispositif de traitement par inhalation selon l'une des revendications précédentes, **caractérisé en ce que** la une ou plusieurs ouvertures de passage d'air respiratoire (23) sont formées en tronçons ou segments d'anneau de cercle.

14. Dispositif de traitement par inhalation selon l'une des revendications précédentes, **caractérisé en ce que** la soupape d'inspiration (20) présente un tronçon de bordure (25), configuré pour assurer la fixation de la soupape d'inspiration (20), en particulier pour l'enserrement entre un paroi (31) du générateur d'aérosol (2) et une paroi (32) de la chambre de mélange (3).

15. Dispositif de traitement par inhalation selon l'une des revendications précédentes, **caractérisé en ce que** la une ou plusieurs ouvertures de passage d'air respiratoire (23) sont prévues de tous côtés autour du passage à aérosol (22).

16. Dispositif de traitement par inhalation selon l'une des revendications précédentes, **caractérisé en ce que** les ouvertures de passage d'air respiratoire (23) sont configurées en s'étendant obliquement, de manière que l'air respiratoire soit guidé en s'éloignant de l'emplacement de fixation de l'élément de soupape (21).

17. Dispositif de traitement par inhalation selon l'une des revendications précédentes, **caractérisé en ce que** la zone des une ou plusieurs ouvertures de passage d'air respiratoire (23) est disposée sensiblement dans un plan commun à celui de la membrane (6).

18. Dispositif de traitement par inhalation selon l'une des revendications précédentes, **caractérisé en ce que** l'élément de soupape (21) est fabriqué en un matériau élastique.

19. Dispositif de traitement par inhalation selon l'une des revendications précédentes, **caractérisé en ce que** la soupape d'inspiration (20) est fabriquée en un matériau élastique.
